Europäisches Patentamt

(19) European Patent Office    (11) Publication number: **0 020 048**

Office européen des brevets    **A1**

(12)    EUROPEAN PATENT APPLICATION

(21) Application number: **80301562.7**    (51) Int. Cl.³: **B 01 J 23/72**

(22) Date of filing: **13.05.80**

(30) Priority: **25.05.79 GB 7918320**

(71) Applicant: **IMPERIAL CHEMICAL INDUSTRIES LIMITED, Imperial Chemical House Millbank, London SW1P 3JF (GB)**

(43) Date of publication of application: **10.12.80 Bulletin 80/25**

(72) Inventor: **Ruddlesden, Jane Frances, 28 Silverdale Close, Frodsham, Cheshire (GB)**
Inventor: **Stewart, Allan, 20 Arran Drive, Frodsham, Cheshire (GB)**

(74) Representative: **Bate, Bernard James et al, Imperial Chemical Industries Limited Legal Department: Patents Thames House North Millbank, London SW1P 4QG (GB)**

(84) Designated Contracting States: **AT BE CH DE FR GB IT LI LU NL SE**

(54) **Novel copper catalyst and process for making it.**

㊼⑦ A hydrogenation catalyst comprising finely divided metallic copper very stable to high temperature use is made by the continuous coprecipitation of copper compounds with hydrated silica from soluble silicate and copper salts with careful control of the pH in the range 4.5–8.0. Subsequent reduction wih hydrogen produces particularly copper (ca. 2–5 nm size) on a porous silica substrate.

ACTORUM AG

1

## Novel Copper Catalyst and Process for making it

This invention relates to catalysts comprising dispersed metallic copper and to processes for making such catalysts.

It is known that catalysts suitable for the hydrogenation of organic compounds may be made by the reduction to the metal of metal salts. The reduction of copper salts or their decomposition products deposited upon a particulate support material, for example, silica or kieselguhr, has been shown to produce effective catalysts. Such catalysts are active for the hydrogenation of organic compounds particularly aliphatic groups, aldehydes and ketones to primary and secondary alcohols. The metallic copper produced by the aforesaid reduction of copper salts may be demonstrated to be present in finely-divided form on the surface of the support material.

However, it has been observed in use that many catalysts although they may be active at low temperature are not stable to use at higher temperatures, for example temperatures above $150^{\circ}$C. The dispersed copper particles tend to agglomerate and the activity of the catalyst decreases. We have now found a method of preparing a catalyst which is much more stable in use at higher temperatures; for example there is a negligible loss in activity when the new catalyst is used in hydrogenation

2

reactions at temperatures up to 250°C.

According to the present invention we provide a process for the production of a catalyst comprising metallic copper dispersed in silica wherein a particulate solid copper compound is precipitated with silica from a mixture of aqueous solutions of a copper salt and a soluble silicate salt and subsequently reduced to dispersed metallic copper in the presence of the precipitated silica characterised in that the final pH of the mixture of aqueous solutions is in the range 4.5 to 8.0.

We prefer the range of pH of the mixture to be from 5.0 to 7.5 especially from 5.5 · to 7.0 when the said precipitation occurs. Although we prefer to carry out the precipitation wholly in the specified range of pH values it is possible to prepare acceptable catalysts by starting with solutions outside the specified pH range and adjusting the pH subsequently, preferably adjusting from a pH greater than 8.0 rather than from a pH lower than 4.5.

It is desirable to produce the precipitated catalyst precursor in as finely-divided a form as possible so that after the reduction in hydrogen the metallic catalyst presents as high a surface area as possible to the reactant molecules. The state of the precursor is important in determining the final catalyst and the methods provided herein ensure a finely-divided form and the result is that very active catalysts may be produced therefrom.

If copper compounds are precipitated from a mixture at a low pH (ca. 2-3) it has been observed that basic copper compounds tend to be formed which on examination are invariably found to consist of large particles, too large to produce a very active copper catalyst even using the most careful reduction techniques. Since the precipitation of a catalyst precursor by adding a precipitating agent (e.g. soluble silicate, hydroxide or carbonate) to an excess of a copper salt in solution (e.g. copper nitrate) would produce a mixture having an initially low pH (<4) we do not recommend such a method because it is likely to produce a catalyst precursor having too large a particle size. If the precipitation must be conducted by adding one solution to the bulk of the other it is preferable to add the copper salt-solution to the bulk of the soluble silicate solution because the pH of the mixture is initially higher ( > 8-9) and reduces as the precipitation proceeds. A better method for the control of pH and hence of the particle size of the precipitate is to .add small quantities of each solution continually, to a stirred vessel, if possible monitoring the quantities added and the pH of the mixture obtained.

The copper salt used in aqueous solution to precipitate the said copper compound may be for example a soluble cupric salt of a medium-to-strong acid and the nitrate has been observed to be especially suitable.

The soluble silicate salt is preferably the salt of an alkali metal but the salt of organic cations may be. used. Sodium silicate or water glass solutions are some of the most readily available soluble silicates and these have been observed to be eminently suitable for effecting the desired precipitation of the copper compound which is to be reduced in order to produce the dispersed copper.

According to another aspect of the present invention we provide a catalyst comprising individual particles of metallic copper having a largest dimension of less than 25 nm and a mean dimension of less than 10 nm for all particles of copper produced by reduction of a copper compound and intimately associated therewith particulate silica co-precipitated with the copper compound from a soluble silicate salt and heat-treated concurrently with the reduction of the copper compound to metallic copper.

Without prejudice to this aspect of the invention we believe that a form of hydrated silica is produced concurrently with the precipitation of the copper compound and that the hydrated silica forms a porous silica carrier for the metallic copper when the precipitated copper compound is reduced in the presence of hydrogen. The copper

4

compound and the silica are coprecipitated by the mixing of the respective aqueous solutions and thus finally the metallic copper and the silica produced during the process are intimately associated together and both form essential elements of the new catalysts. Without prejudice to the invention it is believed that the intimate association of the finely divided metallic copper with this silica (and possibly also the porous nature of the silica) contributes to the excellent stability-in-use of the catalyst. It appears from the evidence provided by electron microscopy and ESCA scanning that the majority of the copper is present as very small particles evenly dispersed in the silica and not mainly on the surface as agglomerates of copper as it is in catalysts prepared by reduction of precipitated copper compounds on pre-formed silica support materials.

In a preferred process within this invention waterglass solutions are used as the soluble silicate salt for example water glass solutions containing a ratio of $Na_2O/SiO_2$ by weight in the range 1:1 to 1:3.

Further NaOH may be added to the silicate solution prior to the precipitation in order to maintain the pH of the reacting mixture with the copper salt in the desired range and during the precipitation NaOH may be added provided that the pH of the mixture remains in the specified range.

The two solutions, namely those of the silicate and the copper salts, should be mixed intimately as rapidly as possible and we prefer to combine a series of small quantities with very efficient stirring as for accurate titrations. We have found that a suitable mode of mixing for preparations on a laboratory scale is to meter quantities of each into a stirred vessel; the initial and subsequent rates of addition of the respective solutions being controlled to establish rapidly and thereafter to maintain the mixture in the stirred vessel as near as possible to a chosen pH value in the range of values specified above. Very good results have been obtained as shown in our Examples from mixing and precipitation experiments lasting for ca. 15 minutes to 1.5 hours. Satisfactory results have also been obtained by slowly adding a solution of a copper salt to a solution of waterglass where much of the precipitation is carried out in the preferred range of pH value i.e. 5.0 to 7.5. This procedure prevents the formation of basic copper salts which can occur at low pH values and spoil the performance of the catalyst.

The precipitation of the total copper salts in solution in the mixture may be completed by the addition of further water soluble alkaline materials if desired after the solutions have been fully mixed. This is an advantageous procedure on economic grounds and we have found that carbonates and bicarbonates of alkali metals and the ammonium ion may be used. However, when waterglass solutions are used for example, it is preferable to complete the precipitation by the subsequent addition of further sodium hydroxide taking care that the final pH does not reach a value greater than 8.0.

The precipitate so produced from processes according to this invention may be treated in a manner conventional for the production of catalysts. After washing and drying the copper compound may be reduced at a temperature in the range $100^{\circ}$ to $500^{\circ}C$ in an atmosphere containing hydrogen, either pure hydrogen or diluted with inert gas conveniently nitrogen, for example in a ratio of ca. 1:10 for the $H_2:N_2$ ratio. Best results with our new catalyst are obtained when the washed, dried precursor is reduced in pure hydrogen at temperatures of up to about $180^{\circ}C$. When these low temperature reduction conditions are used the final catalysts are observed to consist of smaller particles of copper for example, particles having a largest dimension of 10 nm and a mean dimension for all particles of less than 5 nm. The catalyst is then ready for use after reduction by either of these methods.

Catalysts according to this invention have been used effectively for the hydrogenation of sugars to sugar alcohols especially keto sugars, e.g. fructose or sorbose. In the hydrogenation of keto-sugars at least two enantiomorphic or stereoisomeric forms of the corresponding sugar alcohols are possible and not only are the catalysts of this invention very active in promoting such hydrogenations but they also produce a useful excess of one enantiomorphic form of the sugar alcohol,e.g. an excess of mannitol from fructose.

Those skilled in the art of hydrogenation using supported metal catalysts will appreciate that there are many areas of use in which copper catalysts provide reasonable activity but more selectivity when compared to supported metal systems other than copper. For example, in the treatment of olefin and diolefin streams containing acetylene impurities, the acetylenic component may be removed by preferential hydrogenation of the olefins over a copper catalyst. Another example is the hydrogenation of soyabean oil wherein it is desirable to selectively hydrogenate the diene compound (a linolenate ester) in the presence of an olefinic compound (a linoleate ester) and copper copper catalysts have been shown to be useful there. Although normally some hydrogenation of the mono-olefin compound occurs, a copper catalyst provides the highest selectivity of metals tested. The catalysts provided in our present invention offer considerable improvements in activity in these areas of use and wherever selectivity is important, our catalysts give the same or better selectivity as provided by other copper catalysts.

The advantage of the catalysts of this invention over prior art catalysts may be observed over a period of time when using them for such hydrogenation reactions at high temperatures for example at temperatures from $100^{\circ}$C-$350^{\circ}$C. The increased stability may be assessed quantitatively by comparing the level of catalytic activity measured by the yield of hydrogenations at $160^{\circ}$C performed

with the same catalyst over a period of time.

Irrespective of the stability of the catalysts in use, we have observed that the average particle size of metallic copper remains very constant for our new catalysts under thermal treatment up to at least 450°C. Considerably less increase in particle size of the copper particles in reduced catalysts is observed compared with that observed with heat-treatment of prior art catalysts comprising copper on a pre-formed silica support. The continuous heat-treatment of a catalyst according to this invention in streams containing hydrogen at temperatures between 250°C and 320°C over periods of up to 900 hours showed mean particle sizes of only 6-9 nm estimated by X-ray diffraction line-broadening.

It is known that the use of ruthenium catalysts for the hydrogenation of fructose gives approximately equimolar yields of the product hexitols, mannitol and sorbitol. We have found that the incorporation of small amounts of Group VIII metals in the catalysts with copper does not significantly alter the improved selectivity to mannitol that copper has been demonstrated to give and this effect has been described in our copending application (European Application No 79300949.9 published as European Patent Application 0006313).

Catalysts have been prepared by the method of this invention using small amounts of ruthenium, rhodium and nickel compounds together with the copper salts in solution. Thus compounds of these Group VIII metals were precipitated with the copper compounds which formed the catalyst precursors and then on reduction these metals would be dispersed with the copper in the final catalyst. Amounts up to 10% by weight of the Group VIII metal relative to copper are preferable.

Selectivities to mannitol in the hexitol products of fructose reduction were observed to be similar to or slightly lower than those obtained with copper alone. The activity of the catalyst measured by the rate of fructose reduction were not always as high as those for catalysts with copper alone. However, ruthenium when incorporated with copper did cause a substantial increase in activity

7 a

for this hydrogenation over the rate observed for copper alone and the selectivity was equally as good.  Without prejudice to this invention we believe that the normally high activity of ruthenium in hydrogenation catalysis relative to other metals is promoting the activity of the copper catalyst in such a way that the normal non-selective  nature of ruthenium is being obscured as a result of the mode of dispersion of the ruthenium in the copper of the catalyst.

The invention is illustrated by the following examples.

8

Example 1

Two solutions were prepared for use in making a catalyst precursor. One was a solution of 100 g cupric nitrate trihydrate dissolved in deionised water and made up to 500 ml with deionised water. The other was a solution of waterglass made from a more concentrated solution, analysed to contain 5.3% Na and 10.8% Si. The waterglass solution used in the preparation was made by diluting 200 g of this concentrated solution to 500 ml with deionised water.

To carry out the precipitation step, 5 g of Davison 952 silica was suspended in 500 ml of deionised water at room temperature in a large beaker. After calibration an electrode for pH measurement was immersed to the appropriate level in the suspension. Two open glass tubes were positioned with their bases under the level of the liquid in the beaker, and two dropping funnels, one containing the prepared cupric nitrate solution and the other containing the waterglass solution, diluted to 500 ml in the above described manner, were supported so that the effluent from each could drop into a glass tube and run into the suspension in the large beaker, where precipitation could occur. The contents of the beaker were stirred vigorously using an air-powered stirrer mechanism with a stirrer of the type using a half-moon shaped ptfe blade.

The two prepared solutions were added dropwise at the same time to set up and maintain a pH of 5.3 to 6.0 in the contents of the large beaker and to cause the pH to be 5.5 when all the prepared waterglass solution had been added. At this point, about 1.5 hours after the start of the addition of the solutions, the addition of the cupric nitrate solution was stopped, 335 ml having been added into the contents of the large beaker. Throughout the additions deionised water from a wash bottle was used to wash any solids splashed up on the sides of the large beaker back into the mixture. In all, some 300 ml of additional deionised water was used in this way. After allowing the

0020048

9

final mixture about 10 minutes of further stirring, the blue mixture in the large beaker was filtered with suction in a large sintered glass funnel (Pyrex grade 2) and washed thoroughly in the filter with about one litre of deionised water, added in stages after the wet solid had been broken up with a spatular to allow more efficient washing.

The filtered and washed solid was then scraped into a large glass crystallizing dish, which was placed in a vacuum oven. The solid was then dried at about $160^{\circ}C$ in this apparatus while it was pumped by a rotary pump protected by a trap for water which was cooled by liquid nitrogen. This drying process took about 6 hours, after which the product was crushed to yield a powder. The recovered dry solid weighed about 80 g and analysis showed that it contained 29.0% Si, 14.2% Cu and 0.06% Na.

This dry solid which was the catalyst precursor was reduced by heating to $450^{\circ}C$ in a stream of gas composed of 10% by volume of hydrogen in nitrogen for five hours. After reduction the catalyst was ready for use in a hydrogenation reaction. From electron micrographs it was observed that the particles of copper had a mean size of 10 nm the largest observed particles being 24 nm in diameter.

Example 2

The preparation of the catalyst was as described in Example 1, except that no Davison 952 silica was suspended in the deionised water, and 350 ml of the described cupric nitrate solution was required. Analysis showed that the recovered dry solid contained 18.0% Cu, 30.8% Si and 0.05% Na.

Example 3

The preparation of the catalyst was as described in Example 1, except that 5 g of kieselguhr white from BDH was added in place of 5 g Davison 952 silica, and 300 ml of the described cupric nitrate solution was required. Analysis showed the recovered dry solid to contain 20.0% Si, 11.0% Cu and 0.14% Na. The catalyst after reduction as described in Example 1 was used in a hydrogenation reaction: the results of this reaction are presented in Example 12 together with the results of the use of catalysts prepared in Examples 1, 4-8 inclusive and 11.

Example 4

The preparation was as described for Example 1, except that 5 g Ketjen CK 300 alumina was used in place of Davison 952 silica and 410 ml of cupric nitrate solution was required. Analysis showed the recovered dry solid to contain 21.4% Si, 13.8% Cu, 1.2% Al and 0.11% Na.

Example 5

The preparation was as described in Example 1, except that pH was maintained between 6.5 and 7.5 and the final pH was 7. 320 ml of cupric nitrate solution was used in the precipitation. Analysis of the recovered dry solid showed it to contain 28.6% Si, 8.2% Cu and 0.6% Na.

Example 6

The preparation was as described for Example 1, except that pH was maintained as close as possible to pH 6.3 and this was the finishing pH. 335 ml of cupric nitrate solution was used. Analysis showed that the 82.1 g of dry solid recovered contained 30.4% Si, 16.7% Cu and 0.30% Na.

Example 7

The preparation was as described in Example 6, except that no Davison 952 silica was used and 338 ml of cupric nitrate solution was required to conduct the precipitation which in this Example was conducted over ca. 20 minutes instead of 1.5 hours.

Analysis showed that the recovered dry solid contained 28.7% Si, 17.5% Cu and 0.30% Na.

Example 8

The preparation was as described in Example 6, except that 9.8 g of sodium hydroxide pellets were added to and dissolved in the prepared waterglass solution. 486 ml of cupric nitrate solution was required to bring the stirred mixture to pH 6.3 on complete addition of the modified waterglass solution.

11

Analysis of the recovered dry solid showed it to contain 16.7% Cu, 27.3% Si and 0.65% Na.

Example 9

A batch of catalyst was prepared from Davison 952 silica, a solution of copper nitrate and a waterglass solution by the method described in Example 1. The resulting dried material contained 14.3% Cu, 28.9% Si and 0.05% Na.

Batches of approximately 2 g of this material were reduced in a stream of 10% $H_2$ in $N_2$ at the chosen temperature of 450°C.

4.6 g of reduced catalyst was added from the vitreous silica tube in which the reduction had been conducted to a stainless steel autoclave of approximately 1 litre capactiy containing 250 ml of an aqueous solution comprising 125 g D-fructose in deionised water. The autoclave head was placed in position and the autoclave sealed and flushed with hydrogen gas before pressurizing with hydrogen to 1500 p.s.i. g. This vessel was equipped with a "hollow stirrer" stirring mechanism (a Dispersimax Type Agitator) which was run at approximately 1000 rpm in order to ensure efficiency in mixing the contents of the autoclave. After pressurizing, the stirring mechanism was turned on and heating begun. The autoclave reached its reaction temperature of 100°C after approximately 20 minutes, at which time, designated as 0-hour, a sample was taken from the autoclave for analysis and pH measurement. The reaction mixture was sampled by bleeding off from the autoclave, while it was still under pressure, approximately 7 ml of the mixture in two parts. The first was retained and the second analysed by the preparation therefrom of trimethyl silyl derivatives of the reduced sugar alcohol products and unreacted ketoses. The derivatives were analysed by gas/liquid chromatography and the detailed procedure is summarised in the following paragraph.

A sample of the supernatant liquid (5-15 mg dry weight equivalent) was pipetted into a screw-cap septum vial. A solution containing hydroxylamine hydrochloride (25 mg) in AR pyridine (1 ml) was then added through the septum and the solution heated at 70°C for 30 minutes. After cooling to room temperature trimethylsilylimidazole (1 ml) was added, the vial shaken and then allowed to stand at room temperature for a further 30 minutes. 1μ 1 of this solution was then analysed by g.l.c. on a 1.5 m x 3 mm glass column packed with 3% silicone OV-17 on "Chromosorb" W(HP) (80-100 mesh) maintained at 130°C for 10 minutes after injection before a programmed temperature rise of 2°C/min to a final temperature of 210°C.

A pH measurement was also made on this second part.

During the time the sample was taken from the autoclave the stirrer was switched off.

After 2.5 hours at the reaction temperature of 100°C, a further sample was taken for analysis and pH measurement and the autoclave and its contents were allowed to cool before depressurization and removal of the autoclave head. All the remaining reaction mixture and catalyst particles were removed and retained. The head and interior of the autoclave were washed thoroughly with deionised water and the washings combined with the recovered reaction mixture and all the samples which had been taken throughout the reaction.

These combined mixtures were filtered through a Pyrex sinter-funnel to remove the catalyst particles, which themselves were further washed with deionised water to wash through as much carbohydrate as possible.

The colourless filtrate was evaporated to dryness on a rotary evaporator and the remaining material was dried to constant weight in a vacuum oven at 80°C in order to give the recovered carbohydrate figures below. Details of the samples taken at the beginning and end of the period at the reaction temperature of 100°C are given in the Table. Mannitol and sorbitol were the only hexitol products.

| Time (hr) at 100°C | pH of sample | % D-Mannitol in hexitol products | % Conversion (approx) |
|---|---|---|---|
| 0 | 6.3 | 71.6 | 30 |
| 2.5 | 5.5 | 71.1 | 97 |

Recovered carbohydrate (constant weight) = 122.3 g

Example 10

The reaction was carried out as in Example 9 except that 125 g of L-sorbose was used in place of D-fructose, 4.7 g of reduced catalyst was used and the time at the reaction temperature of 100°C was 3 hours.

The following table gives the analyses and pH measurements of samples taken. Sorbitol and iditol were the hexitol products.

| Time (hr) at 100°C | pH of sample | D-glucitol (Sorbitol) as % of hexitols | % Conversion |
|---|---|---|---|
| 0 | 6.7 | - | 17 |
| 3 | 5.3 | 69.5 | 98 |

Recovered carbohydrate (constant weight) = 125.0 g

Example 11

The catalyst preparation was as described in Example 1 except that the catalyst precursor was reduced in a steel tube in a stream of hydrogen. The temperature was raised gradually to the chosen temperature of 180°C over 1.5 hours. It was maintained at this temperature for a further 1.5 hours before cooling to room temperature. The catalyst was then used in the hydrogenation of fructose as described in Example 12 with the results therein shown in tabular form.

14

Example 12

Several reductions of D-fructose were carried out in the manner described in Example 9 except that the catalyst used in each reduction was prepared as described in the relevant Examples. The dry solids obtained from Examples 4, 5, 6, 7 and 8 as catalyst precursors were reduced in a mixture of nitrogen and hydrogen (10%) as described in Example 1 in order to prepare the catalyst. Samples were taken at 30 minute intervals during each 2.5 hour reduction at 100°C and analysed by derivatisation and g.l.c. analysis of the derivatives as described in Example 9. The conversion of fructose to mannitol and sorbitol showed constant selectivity to mannitol of approximately 69% and followed first-order kinetics in fructose disappearance.

The following table shows results obtained in hydrogenations of D-fructose and gives approximate rate constants for the first-order process as taken from the slope of the first order plots of $\ln \left( \frac{a}{a-x} \right)$ against time t. The symbols a and a-x represent the concentrations of fructose initially and at time t respectively, both expressed in the same units of concentration.

| Catalyst from Example | Amount of Reduced Catalyst used g. | Approx. Copper Content in Reduced Catalyst wt% | Experimental Rate Constant hour-1 |
|---|---|---|---|
| 1 | 4.6 | 19 | 1.2 |
| 3 | 3.3 | 20 | 1.1 |
| 4 | 4.0 | 22 | 1.6 |
| 5 | 4.6 | 12 | 1.2 |
| 6 | 5.2 | 21 | 1.9 |
| 7 | 5.2 | 22 | 2.0 |
| 8 | 3.0 | 22 | 1.2 |
| 11 | 1.9 | 19 | 0.5 |

Example 13

50 ml of 3.2 M sodium silicate solution was mixed thoroughly with 500 ml deionised water. 5 g Davison 952 silica was added and mixed. Then a solution of 101 g cupric nitrate trihydrate in 500 ml water and was added dropwise with stirring. Then the mixture was heated to 70°C and a solution of 80 g ammonium bicarbonate in 500 ml deionised water was added dropwise with stirring. The slurry was boiled and stirred for a further 45 minutes. The green solid was filtered off and washed thoroughly with deionised water. Analysis of solid gave Na 0.19%, Cu 32.5% Si 16.6%.

Portions of the above solid were reduced in a stream of hydrogen gas at various temperatures and used in the hydrogenation of invert sugar as shown in the Table below.

Three hydrogenation runs, 17A, 17B and 17C, were carried out and the invert sugar solution was maintained at 120°C for 0.5 hour, then at 140°C for 0.5 hour, then 160°C for 1 hour. Catalyst reduction temperatures, final pH values, and product analyses for each run are given in the table below.

| Run | 17A | 17B | 17C |
|---|---|---|---|
| Catalyst Usage, g. | 5.65 | 5.70 | 5.55 |
| Final pH | 4.8 | 4.5 | 4.8 |
| Catalyst reduction temp °C | 325 | 225 | 200 |
| Product analysis | | | |
| Sorbitol | 61.4 | 63.3 | 63.0 |
| Mannitol | 31.0 | 32.3 | 31.8 |
| Total Sugar | 0.6 | 0.5 | 0.3 |
| Reducing Sugar | 0.25 | 0.11 | 0.03 |

Example 14

A catalyst was prepared by the method described in Example 1 except that the waterglass solution used was more concentrated. 200g of the concentrated solution (5.3% Na and 10.8% Si) was diluted only to 250 ml with deionised water. The pH was controlled during the precipitation mostly at a pH of 6.3 and the final pH was measured as 5.5. The filtered, washed and dried solid contained 25.3% Si, 14.5% Cu and 0.12% Na. 3.7g of this solid was reduced in a gaseous mixture of 10% hydrogen in nitrogen for 6 hours and the catalyst thus produced gave an experimental rate constant of 1.63 h$^{-1}$ (defined in Example 14) when used for the hydrogenation of fructose under the same conditions as those in Example 12.

Example 15

A catalyst was prepared by the method described in Example 1 except that the waterglass solution used was less concentrated. 200g of the concentrated solution (5.3% Na and 10.8% Si) was diluted to 1000 ml with deionised water. The pH was controlled mostly at pH of 6.3 during the precipitation and the final pH was measured as 5.6.

2.1g of the dried solid from this precipitation was reduced at 450°C in a gaseous mixture of 10% hydrogen in nitrogen for 6 hours and the catalyst thus produced gave an experimental rate constant of 0.86 h$^{-1}$ (defined in Example 12) when used for the hydrogenation of fructose under the same conditions as those in Example 12.

Example 16

Two solutions were prepared for use in making a catalyst precursor. One was a solution of 66 g cupric nitrate trihydrate and 1.2 g ruthenium trichloride trihydrate dissolved in deionised water and made up to 350 ml with deionised water. The other was a solution of waterglass made from a more concentrated solution, analysed to contain 5.3% Na and 10.8% Si. The waterglass solution used in the precipitation was made by diluting 200 g of this concentrated solution to 500 ml with deionised water.

To carry out the precipitation step, 500 ml of deionised water was placed in a large beaker at room temperature. After calibration an electrode for pH measurement was immersed to the appropriate level in the suspension. Two open glass tubes were positioned with their bases under the level of the liquid in the beaker, and two dropping funnels, one containing the prepared solution of cupric and ruthenium salts and the other containing the waterglass solution, diluted to 500 ml in the above described manner, were supported so that the effluent from each could drop into a glass tube and run into the suspension in the large beaker, where precipitation could occur. The contents of the beaker were stirred vigorously using an air-powered stirrer mechanism with a stirrer of the type using a half-moon shaped ptfe blade.

The two prepared solutions were added dropwise at the same time to set up and maintain a pH of 6.3 in the contents of the large beaker and when all solutions had been added the final pH was 5.8, about 0.5 hours after the the start of the addition of the solutions. Throughout the additions deionised water from a wash bottle was used to wash any solids splashed up on the sides of the large beaker back into the mixture. In all, some 300 ml of additional deionised water was used in this way. After allowing the final mixture about 10 minutes of further stirring, the mixture in the large beaker was filtered with suction in a large sintered glass funnel (Pyrex grade 2) and washed thoroughly in the filter with about one litre of deionised water, added in stages after the dark green wet solid had been broken up with a spatula to allow more efficient washing.

The filtered and washed solid was then scraped into a large glass crystallizing dish, which was placed in a vacuum oven. The solid was then dried at about 160°C in this apparatus while it was pumped by a rotary pump protected by a trap for water which was cooled by liquid nitrogen. This drying process took about 6 hours, after which the product was crushed to yield a powder. The recovery dry solid was analysed and no chlorine was detected. The analysis showed that it contained 0.4 Ru, 12.79 Cu, 24% Si and 0.1% Na.

This dry solid which was the catalyst precursor was reduced by heating to 450°C in a stream of gas composed of 10% by volume of hydrogen in nitrogen for five hours. After reduction the catalyst was ready for use in a fructose

18

hydrogenation reaction which was carried out at 100$^{\circ}$C and 1500 psig hydrogen under the conditions shown in Example 9. For 2.9g of reduced catalyst the experimental rate constant was 2.18 h$^{-1}$ as defined in Example 12. The pH of the hydrogenation mixture was 6.94 at the beginning of the reaction and after 2 hours was found to have dropped to 6.0. The percentage of mannitol in the hexitol products of this reaction was approximately 69% comparable to the selectivity of copper as the sole metal in the catalyst.

Example 17

An experiment was conducted as in Example 16 except that 1.2g rhodium trichloride trihydrate was used in place of the similar ruthenium salt and a catalyst was produced and used in the same way. A trace of chloride was observed in the analysis of the dried solid at a level of 0.21% chlorine by weight. Use of this catalyst gave mannitol levels which were measured as 66% of the hexitol products and this is slightly lower than that found with equivalent catalysts using copper as the sole metal. The activity of the catalyst also appeared to be lower which may have been the result of the presence of chloride during the reduction step in producing the catalyst.

What we claim is:

1.      A process for the production of a catalyst comprising metallic copper dispersed in silica wherein a particulate solid copper compound is precipitated with silica from a mixture of aqueous solutions of a copper salt and a soluble silicate salt and subsequently reduced to dispersed metallic copper in the presence of the precipitated silica characterised in that the final pH of the mixture of aqueous solutions is in the range 4.5 to 8.0

2.      A process as claimed in claim 1 wherein the pH of the mixture is from 5.0 to 7.5 when the precipitation occurs.

3.      A process as claimed in claim 2 wherein the pH of the mixture is from 5.0 to 7.0 when the precipitation occurs.

4.      A process as claimed in any one of the preceding claims wherein a series of small quantities of the solutions of both the copper salt and the silicate salt are combined in a vessel with efficient stirring.

5.      A process as claimed in claim 4 wherein the quantities of the solutions of copper salt and silicate salt brought together are controlled to establish rapidly and thereafter maintain the pH of the mixture in the stirred vessel within the range 5.0 to 7.5.

6.      A process as claimed in any one of the claims 1-5 wherein the soluble silicate salt is the salt of an alkali metal.

7.      A process as claimed in claim 6 wherein the alkali metal is sodium.

8.      A process as claimed in any one of the preceding claims wherein the copper compound is reduced in the presence of hydrogen.

9.      A process as claimed in claim 8 wherein the copper compound is dried and powdered prior to reduction.

10.     A process as claimed in claim 8 or 9 wherein the reduction is carried out in the temperature range 100-500°C.

11.     A process as claimed in claim 10 wherein the reduction is carried out in pure hydrogen at temperatures of up to about 180°C.

12.    A process as claimed in claim 10 wherein the reduction is carried out in a mixture of nitrogen and hydrogen at a temperature in the range 180°C-500°C.

13.    A process as claimed in any one of the preceding claims wherein the precipitation of the copper compound is completed by the addition of further water soluble alkaline materials after the solutions have been fully mixed.

14.    A process as claimed in claim 13 wherein the water soluble alkaline materials are the carbonates and bicarbonates of alkali metals or of the ammonium ion.

15.    A process as claimed in claim 13 wherein a solution of water glass is used as the silicate solution and the precipitation is completed by the subsequent adding of sodium hydroxide.

16.    A process as claimed in any one of claims 1-15 wherein a compound of a Group VIII metal is present in solution with the copper salt when the precipitation occurs.

17.    A process as claimed in claim 16 wherein the Group VIII metal is ruthenium.

18.    A catalyst comprising individual particles of metallic copper having a largest dimension of less than 25 nm and a mean dimension of less than 10 nm for all particles of copper produced by the reduction of a particulate solid copper compound and intimately associated therewith particulate silica co-precipitated with the copper compound from a soluble silicate salt and heat-treated concurrently with the reduction of the copper compound to metallic copper.

19.    A catalyst as claimed in claim 18 wherein the copper particles have a largest dimension of less than 10 nm and a mean dimension of less than 5 nm.

20.    A catalyst as claimed in claim 18 or 19 wherein the copper compound was a finely-divided solid precipitated from a mixture of aqueous solutions of a copper salt and of a soluble silicate salt.

21.    A catalyst as claimed in claim 20 wherein the precipitated solid was produced from a mixture of solutions maintained in the pH range from 5.0 to 7.5.

22.    A catalyst as claimed in any one of the claims 16-19 wherein the soluble silicate salt is an alkali metal silicate.

23.    A catalyst as claimed in claim 22 wherein the alkali metal is sodium.

24.    A catalyst as claimed in any one of claims 18-23 wherein a Group VIII metal is present with the copper at a concentration of up to 10% by weight relative to the weight of copper.

25.    A catalyst as claimed in claim 24 wherein the Group VIII metal is ruthenium.

26.    A catalyst as claimed in claim 18 produced by a process as claimed in any one of claims 1-17.

B J BATE
AGENT FOR THE APPLICANTS

European Patent Office

**EUROPEAN SEARCH REPORT**

Application number

EP 80 30 1562

## DOCUMENTS CONSIDERED TO BE RELEVANT

| Category | Citation of document with indication, where appropriate, of relevant passages | Relevant to claim |
|---|---|---|
| X | DE - C - 845 040 (THE DISTILLERS COMPANY)<br><br>* Claims 1,10,11,12; examples 1 and 2 * | 1-7 |
| | US - A - 1 823 865 (LLOYD CLAYTON SWALLEN)<br><br>* Page 1, lines 30 to 52; and 87 to 97 * | 1,4, 6-11 |
| | DE - B - 1 228 740 (ASAHIDENKA)<br><br>* Claims 1 and 3; example 1 * | 1-5, 16,17 |
| | DE - B - 2 538 253 (RUHRCHEMIE)<br><br>* Claims; examples 4 and 6 * | 1-8, 10, 12-15 |
| | FR - A - 896 762 (SOCIETE POUR L'INDUSTRIE CHIMIQUE)<br><br>* Claim 1 * | 1,15 |

**CLASSIFICATION OF THE APPLICATION (Int. Cl. 3)**

B 01 J 23/72

**TECHNICAL FIELDS SEARCHED (Int.Cl. 3)**

B 01 J 23/72
23/70

**CATEGORY OF CITED DOCUMENTS**

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

The present search report has been drawn up for all claims

| Place of search | Date of completion of the search | Examiner |
|---|---|---|
| The Hague | 12.09.1980 | THION |

EPO Form 1503.1 06.78